# EUROPEAN PATENT SPECIFICATION

(11) **EP 3 329 959 B1**
(45) Date of publication and mention of the grant of the patent: **26.08.2020**
(21) Application number: 16830123.2
(22) Date of filing: 11.05.2016
(51) Int. Cl.: A61M 25/10, A61M 25/098, A61B 1/018, A61B 17/00

(54) **TREATMENT INSTRUMENT FOR ENDOSCOPE**
BEHANDLUNGSINSTRUMENT FÜR ENDOSKOP
INSTRUMENT DE TRAITEMENT POUR ENDOSCOPE

(30) Priority: 28.07.2015 JP 2015148480
(43) Date of publication of application: 06.06.2018
(73) Proprietor: Olympus Corporation, Hachioji-shi, Tokyo 192-8507 (JP)
(72) Inventor: ONISHI Koji, Hachioji-shi, Tokyo 192-8507 (JP); MIYAKE Koji, Hachioji-shi, Tokyo 192-8507 (JP); FUKUSHIMA Norichika, Hachioji-shi, Tokyo 192-8507 (JP); YUASA Masaru, Hachioji-shi, Tokyo 192-8507 (JP)
(74) Representative: Schicker, Silvia
(86) International application number: PCT/JP2016/063985
(87) International publication number: WO 2017/018018

(56) References cited:
- EP-A1- 1 884 187
- WO-A1-01/12255
- WO-A1-2009/066330
- JP-A- 2000 509 304
- JP-A- 2004 525 704
- JP-A- 2006 239 156
- JP-A- 2014 124 265
- JP-A- 2014 188 205
- JP-A- 2014 509 218
- JP-A- 2015 518 776
- US-A- 4 346 698
- US-A1- 2003 225 312
- US-A1- 2011 190 867
- US-A1- 2013 261 655

## Description

### [Technical Field]

The present invention relates to a treatment instrument for an endoscope used when performing a dilation treatment on a stenosed part or an occluded part in a luminal organ of a living body.

Priority is claimed on Japanese Patent Application No. 2015-148480 (JP20150148480), filed July 28, 2015.

### [Background Art]

Conventionally, a procedure for performing a dilation treatment or the like of a stenosed part or an occluded part (hereinafter referred to as "stenosed part or the like") of the digestive tract while using an endoscope is performed. In such a procedure, for example, a treatment instrument for an endoscope equipped with a balloon is used. Specifically, the endoscope and the treatment instrument for the endoscope are inserted into the luminal organ of the living body together, and the balloon is inflated while the balloon is inserted into the stenosed part or the like to dilate the stenosed part or the like. When the treatment instrument for an endoscope is disposed to dilate the stenosed part in the luminal organ of the living body in this manner, the balloon slides against the stenosed part or the like while the balloon is inflated, and the balloon may become detached from the part to be dilated. In this case, it is necessary for a surgeon to temporarily deflate the balloon and perform positioning of the balloon again, which makes the operation complicated.

Therefore, in order to prevent the balloon from slipping with respect to the stenosed part or the like in a state of being inflated, a treatment instrument for an endoscope using a balloon having a small-diameter portion between a distal end portion and a proximal end portion in a state of being inflated has been proposed (see, for example, Patent Literatures 1 to 3).

As a further example, Patent Literature 4 discloses a balloon dilator including a balloon which is inflated by supplying a fluid through a fluid supply conduit. When the fluid is supplied to dilate the balloon, a central portion of the balloon is expanded so as to have a minimum expansion diameter, and the internal pressure of the balloon is maintained until a constriction portion, located at the central portion, is appropriately expanded. At this time, the internal pressure is also applied to sloped portions formed at the proximal end side and at the distal end side of the balloon. The thickness of the sloped portions gradually becomes thinner from the central portion side of toward the supply conduit side.

### [Citation List]

### [Patent Literature]

[Patent Literature 1]
   PCT International Publication No. WO2010/042869
[Patent Literature 2]
   Japanese Unexamined Patent Application, First Publication No. 2010-4915 (JP2010004915A)
[Patent Literature 3]
   PCT International Publication No. WO00/057945
[Patent Literature 4]
   Japanese Patent Application Publication No. JP2006239156A

### [Summary of Invention]

### [Technical Problem]

In order to minimize the slippage of the balloon introduced into the digestive tract so that its entire length becomes longer than the stenosed part in the digestive tract and its distal and proximal ends are located outside a range of constriction or the like, a central part of the balloon preferably has a thin shape at an initial stage in a state of being inflated. On the other hand, in order to sufficiently dilate the stenosed part or the like, it is desirable that the diameter of the central part of the balloon finally also expands to the same extent as the end portion.

In the balloons disclosed in Patent Literatures 1 to 3, balloons in which properties or thicknesses of materials vary depending on the parts of the balloon are manufactured, and the pressure resistance performance varies. Therefore, the manufacturing process is complicated and the costs are high. Further, in the balloons disclosed in Patent Literatures 1 to 3, it is difficult to form a film of the balloon with a uniform strength, and there is a risk of damage easily occurring in a part with a small thickness or a part with low values for physical properties such as tensile properties, due to a tensile force applied in a state of being inflated. Furthermore, the dilation force of the central part of the balloon is smaller than that of the end portion, and the stenosed part or the like may not be able to be sufficiently dilated.

In view of the above circumstances, an object of the present invention is to provide a treatment instrument for an endoscope capable of stably inflating a balloon at an appropriate position in a luminal organ of a living body, and appropriately dilating a stenosed part and an occluded part.

### [Solution to Problem]

A treatment instrument for an endoscope according to the present invention includes the features of claim 1. A treatment instrument for an endoscope may be equipped with a sheath; and a balloon which is provided at a distal end of the sheath, the balloon being configured to be expandable to an unfolded inflated shape from a folded initial shape by supplying a fluid, the balloon having a first region and a second region provided at both end portions in a longitudinal direction, and an intermediate section provided between the first region and the second region.

According to the invention, in the initial state of the balloon, an amount of residual strain of the intermediate section is larger than the amount of residual strain of the first region and the amount of residual strain of the second region. When an internal pressure of the balloon has a first internal pressure value, the first region and the second region are unfolded faster than the intermediate section, thereby having a greater diameter than the intermediate section. When the internal pressure of the balloon has a second internal pressure value greater than the first internal pressure value, the first region, the second region, and the intermediate section are unfolded to have the inflated shape, and the diameter of the intermediate section and the diameters of the first region and the second region are substantially the same.

The balloon may be folded to have a plurality of wing sections protruding radially outward in the initial shape, and the wing sections may be folded by being wound around an axis of the balloon.

According to the present invention, the balloon is configured such that, when the internal pressure is greater than a third internal pressure value greater than the second internal pressure value, a material forming the balloon stretches and expands to have a diameter greater than that at the second internal pressure value.

The balloon may be configured such that an outer diameter increases up to the second internal pressure value, according to the inflation due to the progress of the unfolding rather than the expansion due to the stretching of the balloon material, and at an internal pressure greater than the third internal pressure value, the outer diameter increases, according to the expansion due to the stretching of the balloon material rather than the inflation due to the progress of the unfolding.

In the initial shape, the outer diameter of the intermediate section may be smaller than the outer diameters of the first region and the second region.

The treatment instrument for the endoscope may further include markers provided at boundaries between the intermediate section, the first region, and the second region.

The marker may be configured to be visible under an endoscope or X-ray fluoroscopy.

### [Advantageous Effects of Invention]

According to the treatment instrument for the endoscope of the present invention, it is possible to stably inflate the balloon at an appropriate position in the luminal organ of the living body and appropriately expand a stenosed part and an occluded part.

### [Brief Description of Drawings]

Fig. 1 is a cross-sectional view illustrating a treatment instrument for an endoscope according to an embodiment of the present invention.
Fig. 2 is a diagram illustrating an initial shape of a balloon in the treatment instrument for the endoscope according to an embodiment of the present invention.
Fig. 3 is a diagram illustrating an example of a procedure of forming the initial shape of the balloon in the treatment instrument for the endoscope according to an embodiment of the present invention.
Fig. 4 is a diagram illustrating an example of a procedure of forming an initial shape of the balloon in a treatment instrument for the endoscope according to an embodiment of the present invention.
Fig. 5 is a diagram illustrating an example of a procedure of forming the initial shape of the balloon in the treatment instrument for the endoscope according to an embodiment of the present invention.
Fig. 6 is a diagram illustrating an example of a procedure of forming the initial shape of the balloon in the treatment instrument for the endoscope according to an embodiment of the present invention.
Fig. 7 is a diagram illustrating one operation at the time of use of the treatment instrument for the endoscope according to an embodiment of the present invention.
Fig. 8 is a graph illustrating a relationship between an inner pressure and an outer diameter of the balloon in the treatment instrument for the endoscope according to an embodiment of the present invention.
Fig. 9 is a diagram illustrating a balloon at the first internal pressure value of the treatment instrument for the endoscope according to an embodiment of the present invention.
Fig. 10 is a diagram illustrating a balloon at a second internal pressure value of the treatment instrument for the endoscope according to an embodiment of the present invention.
Fig. 11 is an enlarged cross-sectional view illustrating part of the balloon in the treatment instrument for the endoscope according to an embodiment of the present invention.
Fig. 12 is a diagram illustrating a modified example of a distal end portion of the treatment instrument for the endoscope according to an embodiment of the present invention.
Fig. 13 is a diagram illustrating a modified example of the distal end portion of the treatment instrument for the endoscope according to an embodiment of the present invention.

### [Description of Embodiments]

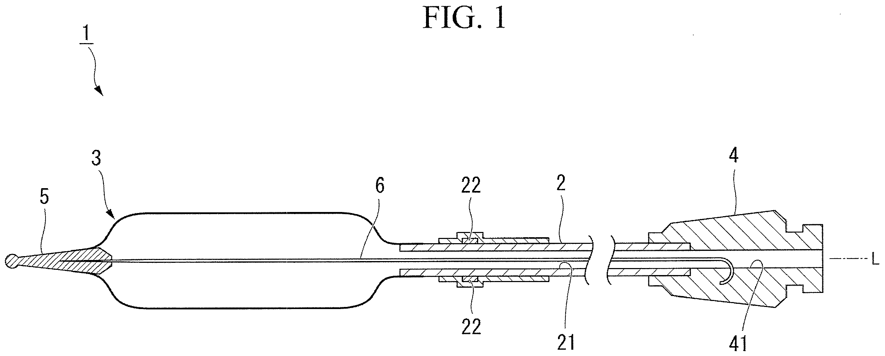

An embodiment of the present invention will be described with reference to Figs. 1 to 10. Fig. 1 is a cross-sectional view illustrating a treatment instrument for the endoscope 1 according to this embodiment. The treatment instrument for the endoscope 1 is equipped with a sheath 2, a balloon 3, a connecting section 4, a distal end tip 5, and a stylet 6.

The sheath 2 is a member which has a lumen 21, is long in a direction of a longitudinal axis L, and has flexibility. A proximal end portion of the balloon 3 is tightly connected to the distal end portion of the sheath 2. The connecting section 4 is provided at the proximal end portion of the sheath 2. The connecting section 4 has a communication passage 41, which allows communication from the distal end to the proximal end along the longitudinal axis L, formed therein. A lumen 21 of the sheath 2 communicates with the interior of the balloon 3 and a communication passage 41 of the connecting section 4. Therefore, the balloon 3 can be inflated, by supplying fluid to the balloon 3 via the communication passage 41 and the lumen 21, using a syringe or the like connected to the connecting section 4 or the like.

At the distal end portion of the sheath 2, a marker 22 capable of being checked under X-ray fluoroscopy is provided.

The balloon 3 is a bag-like member made up of, for example, a transparent film (for example, PEBAX (registered trademark) manufactured by ARKEMA Co.) made of polyamide resin.

The balloon 3 is folded by forming folds such that a plurality of wing sections extending in the longitudinal direction are formed after forming the material into a substantially cylindrical shape. Hereinafter, a shape in which a diameter is reduced by this folding is referred to as an "initial shape" of the balloon 3. Details of the initial shape will be described later.

When the fluid is injected, the balloon 3 is deployed while unfolding by the wing section spreading, and inflates to a substantially cylindrical shape. In this way, a shape after inflating substantially only by unfolding is hereinafter referred to as the "inflated shape" of the balloon 3. By removing the fluid injected into the inflated balloon 3, the balloon 3 can fold again and deflate in a dimension in a radial direction. When the internal pressure of the balloon 3 having the inflated shape becomes a predetermined value or more, the balloon 3 expands to increase in diameter, while the constituent film stretches. That is, the balloon 3 is a so-called semi-compliant balloon.

The distal end tip 5 is provided at the distal end of the treatment instrument for the endoscope 1. The distal end tip 5 is a substantially conical member extending in the direction of the longitudinal axis L, and its distal end portion is formed in a spherical shape in order to prevent damage to tissue when inserted into a body cavity.

The distal end portion of the balloon 3 is tightly fixed to the proximal end portion of the distal end tip 5.

The stylet 6 is a shaft member, is inserted through the inside of the balloon 3, and extends along the longitudinal axis L from the distal end to the proximal end of the balloon 3. The distal end portion of the stylet 6 is connected to the proximal end of the distal end tip 5. The stylet 6 extends through the interior of the balloon 3, the lumen 21 of the sheath 2, and the communication passage 41 of the connecting section 4 and is fixed to the inner wall of the communication passage 41 of the connecting section 4. The stylet 6 is made of, for example, stainless steel, a nickel-titanium alloy or the like.

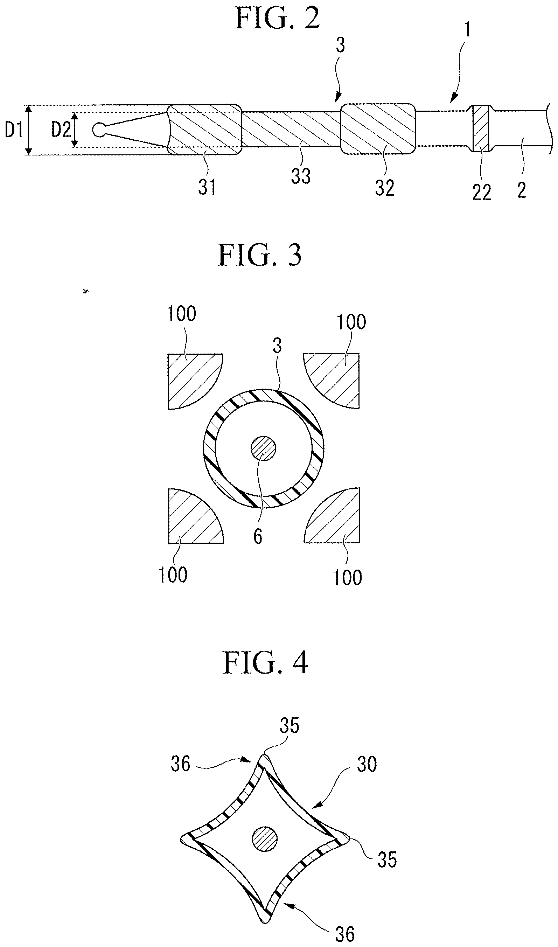

FIG. 2 illustrates the initial shape which is the shape of the balloon 3 before inflation. In the initial configuration, the balloon 3 has a first region 31 adjacent to the distal end tip 5, a second region 32 adjacent to the sheath 2, and an intermediate section 33 between the first region 31 and the second region 32. An outer diameter D1 of the first region 31 and the second region 32 located at both ends in the longitudinal direction (the same as an axial direction of the balloon 3) of the balloon 3 is larger than an outer diameter D2 of the intermediate section 33.

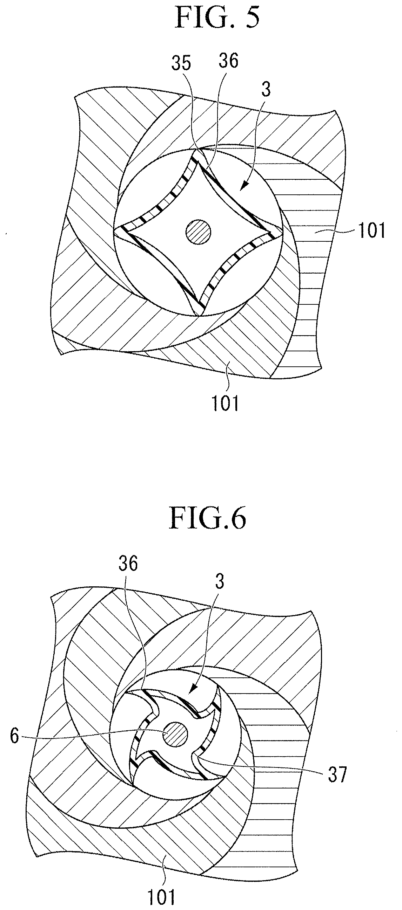

A method of a folding process for forming the aforementioned initial shape will be described. The folding process of the balloon 3 is performed by combining the folding process illustrated in Figs. 3 and 4 and the winding process illustrated in Figs. 5 and 6.

First, as illustrated in Fig. 3, a plurality of folding members 100 are brought into close contact with each other from the outer side of the balloon 3 in the radial direction. Then, part of the balloon 3 is sandwiched between the folding members 100 and bent. Thus, as illustrated in Fig. 4, a folding line 35 extending in the longitudinal direction of the balloon 3 is formed, and the balloon 3 has a plurality of wing sections 36 formed to protrude radially outward with the folding line 35 as a ridge line. The number of wing sections 36 to be formed can be appropriately set by changing the number of folding members 100.

Next, as illustrated in Fig. 5, a plurality of diaphragm members 101 are brought into close contact with each other from the radially outer side of the balloon 3 having the wing section 36 formed thereon. By combining the plurality of the diaphragm members 101, a columnar internal space can be formed at the center portion thereof, and by appropriately relatively moving the plurality of diaphragm members 101, it is possible to change the dimension of the internal space in the radial direction.

When the plurality of diaphragm members 101 are relatively moved in the state of the balloon 3 being disposed in the internal space and the radial dimension of the internal space is gradually reduced, the diaphragm member 101 and the ridge line of the wing section 36 are first brought into contact with each other. Thereafter, due to friction between the diaphragm member 101 and the wing section 36, the ridge line of the wing section 36 moves in the circumferential direction of the balloon 3, and the protruding direction of the wing section 36 is inclined in the circumferential direction. Thereafter, as the radial dimension of the internal space is reduced, as illustrated in Fig. 6, the wing section 36 is wound around the axis of the balloon 3 about the stylet 6 to provide curling. At this time, a plurality of valley sections 37 are formed to protrude radially inward at a base side of the wing section 36. The outer diameter of the curled balloon 3 is substantially the same as the inner diameter of the internal space formed by the diaphragm member 101. Accordingly, a pair of diaphragm members 101 having sizes corresponding to the first region 31, the second region 32, and the intermediate section 33 are prepared, respectively, and the diaphragm member 101 disposed around the first region 31 and the second region 32 is relatively moved until the inner diameter of the internal space becomes D1, and the diaphragm member 101 disposed around the intermediate section 33 is relatively moved until the inner diameter of the internal space becomes D2. Accordingly, it is possible to form the balloon 3 having the initial shape illustrated in Fig. 2.

The above-described forming method of the initial shape is an example, and the method for forming the balloon according to the present embodiment is not limited thereto.

In the balloon 3 having this initial shape, the amount of residual strain differs between the first region 31, the second region 32 and the intermediate section 33, due to a difference in amount of deformation of the film caused by the folding process. In the present specification, the "amount of residual strain" means the total amount of residual strain within a predetermined unit length range in the axial direction of the balloon. When a certain region has a length equal to or longer than the unit length in the axial direction, the amount of residual strain per unit length calculated by averaging the amount of residual strain of each part is taken as the amount of residual strain in the region.

In the balloon 3 having the initial shape, the residual strain exclusively occurs in the portion of the folding line 35, which is the top portion of the wing section 36, and the valley section 37 bent so as to be convex toward the stylet 6 at the middle between two adjacent wing sections 36. As the amount of deformation occurring in the film constituting the balloon 3 due to the folding process increases, the residual strain generated in the folding line 35 and the valley section 37 increases. Accordingly, the balloon 3 according to the present embodiment is configured so that the amount of residual strain is larger in the intermediate section 33 than in the first region 31 and the second region 32.

In the aforementioned method for forming the initial shape, it is possible to adjust the amount of deformation of the balloon 3, for example, by adjusting the movement distance of the diaphragm member 101 inward in the radial direction of the balloon at the time of the winding process. Therefore, in the balloon 3 according to the present embodiment, at the time of forming the initial shape, by setting the movement distance of the diaphragm member 101 disposed around the intermediate section 33 to be larger than the movement distance of the diaphragm member 101 disposed around the first region 31 and the second region 32, the amount of deformation of the intermediate section 33 is set to be larger than the amounts of deformation of the first region 31 and the second region 32. Therefore, the amount of residual strain in the intermediate section 33 can be set to be larger than the amount of residual strain in the first region 31 and the second region 32.

The method for adjusting the amount of residual strain is not limited thereto, and for example, by setting the amount of driving force of the diaphragm member 101 at the time of winding process, or the amount of driving force or movement distance of the folding member 100 at the time of folding process, the amount of residual strain may be set to be larger at the intermediate section 33 than at the first region 31 and the second region 32. Further, the amount of residual strain may be adjusted by performing a heat treatment on the balloon 3 at the time of winding process or the folding processing.

An operation at the time of use of the treatment instrument for the endoscope 1 configured as described above will be described.

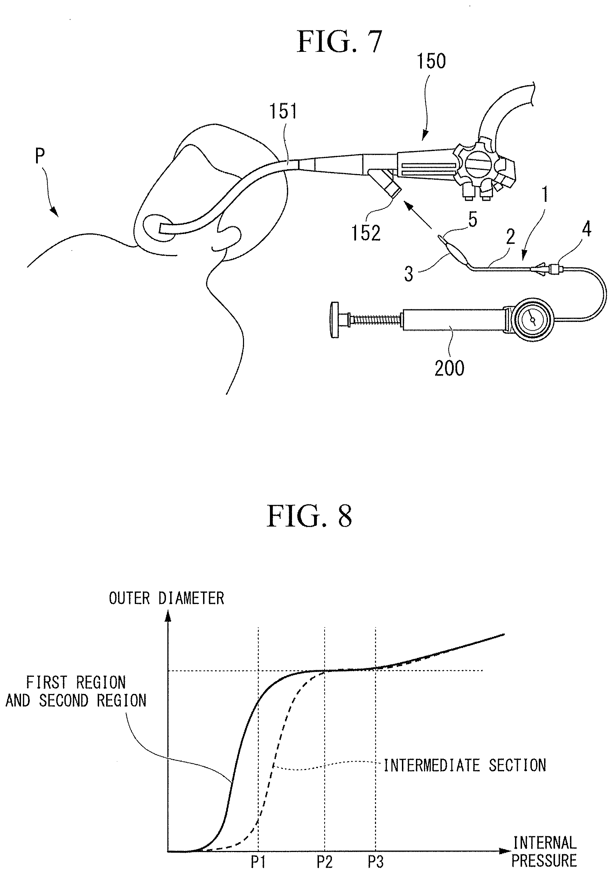

The treatment instrument for the endoscope 1 is introduced into the body of the patient P via a channel provided in the insertion portion 151 of the endoscope 150. As illustrated in Fig. 7, the user connects an inflator 200 to the connecting section 4, and inserts the inflator into the insertion portion 151 from a forceps port 152 of the endoscope 150. Thereafter, the endoscope 150 is inserted into the body of the patient P, and the distal end of the endoscope 150 is moved forward to a region in which the dilation procedure is performed, for example, to the vicinity of a predetermined part of the esophagus. Connection between the treatment instrument for the endoscope 1 and the inflator 200 or insertion of the treatment instrument for the endoscope 1 into the endoscope 150 may be performed after the endoscope 150 is inserted into the body of the patient P.

While observing a target site to be subjected to the dilation procedure with the endoscope 150, the user causes the instrument for an endoscope 1 to protrude from the endoscope 150 and inserts the distal end tip 5 into the target site. The user further moves the treatment instrument for the endoscope 1 forward and holds the treatment instrument for the endoscope 1 so that the balloon 3 breaks through the target site, that is, the distal end portion and the proximal end portion of the balloon 3 are located on the distal side and the proximal side with respect to the target site, respectively.

The user actuates the inflator 200 to supply a fluid such as water or air to the balloon 3. The balloon 3 inflates while the internal pressure is raised by the supplied fluid. Since the amount of residual strain of the intermediate section 33 is larger than that of the first region 31 and the second region 32, a larger force is required such that the folding line 35 and the valley section 37 linearly stretch and the wing section 36 spreads.

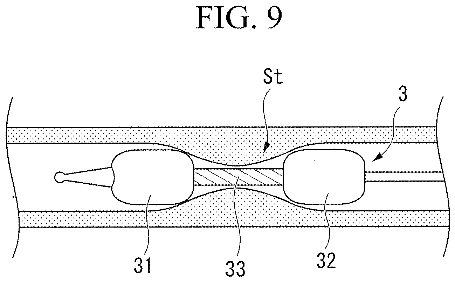

Fig. 8 is a graph illustrating a relationship between the internal pressure of the balloon 3 and the outer diameters of the first region 31, the second region 32, and the intermediate section 33. In a state in which the internal pressure of the balloon 3 has reached a predetermined first internal pressure value PI due to the supply of the fluid, in the first region 31 and the second region 32, unfolding has progressed faster than at the intermediate section 33. On the other hand, since the progress of unfolding of the intermediate section 33 is slow, the diameter of the intermediate section 33 is smaller than that of the first region 31 and the second region 32, and the balloon 3 overall has a dumbbell shape as illustrated in Fig. 9. Therefore, even if the balloon 3 slips due to mucus or the like on the surface of the luminal organ, the first region 31 and the second region 32 serve as an anchor to suppress the movement, and thereby a situation such as detachment of the balloon 3 from the target site St is suitably prevented.

In Fig. 9, the shape of the intermediate section 33 illustrates the same state as the initial shape, but this is an example, and it is obvious that the shape may be a state when the intermediate section starts to inflate.

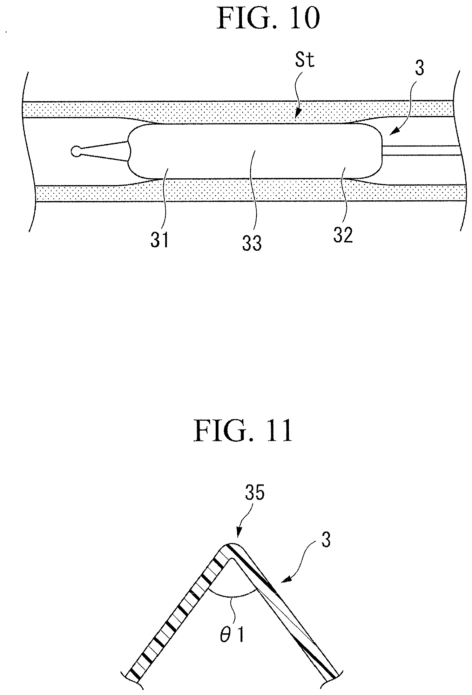

When the internal pressure of the balloon 3 reaches the second internal pressure value P2, which is higher than the first internal pressure value, the folding is released at all of the first region 31, the second region 32, and the intermediate section 33, and the balloon 3 is restored to almost a substantially cylindrical shape (inflated shape) before the folding process as illustrated in Fig. 10. At this time, the radial dimensions of the first region 31, the second region 32, and the intermediate section 33 are the same or substantially the same. Since the intermediate section 33 inflates to substantially the same diameter as the first region 31 and the second region 32, the target site St can be sufficiently dilated.

At the second internal pressure value P2, the film itself forming the balloon 3 is hardly stretched. Here, since the balloon 3 is a semi-compliant type, by setting the internal pressure to be larger than a third internal pressure value P3 which is higher than the second internal pressure value as necessary, the whole of the balloon 3 is further inflated, while stretching the material, and a larger dilation force can be applied to the target site St.

In more detail, since the material forming the balloon 3 itself hardly stretches until the internal pressure of the balloon 3 reaches the second internal pressure value P2, the balloon 3 inflates exclusively depending on the progress of unfolding, and the outer diameters of the first region 31, the second region 32, and the intermediate section 33 increase.

Since the folding is almost released after the internal pressure of the balloon 3 reaches the second internal pressure value P2, even if the internal pressure rises, almost no increase in the external diameter occurs. When the internal pressure of the balloon 3 rises further and becomes larger than the third internal pressure value P3, the film material forming the balloon 3 starts to stretch. However, since expansion due to the progress of unfolding hardly occurs, the outer diameters of the first region 31, the second region 32, and the intermediate section 33 increase exclusively depending on the stretching of the film material.

As described above, according to the treatment instrument for the endoscope 1 of the present embodiment, the amount of residual strain of the intermediate section 33 in the balloon 3 is set to be larger than the amount of residual strain of the first region 31 and the second region 32 disposed with the intermediate section 33 interposed therebetween. As a result, at the first internal pressure value P1, a dumbbell shape in which the first region 31 and the second region 32 have inflated to have an outer diameter larger than that of the intermediate section 33 is obtained, and it is possible to suitably prevent the balloon from being detached or displaced from the target site St during the treatment process on the target site St.

Further, at the second internal pressure value P2, the intermediate section 33 can be inflated to substantially the same diameter as the first region 31 and the second region 32, and the target site St can be sufficiently dilated.

As a result, prevention of misalignment with respect to the target site and sufficient expansion of the target site are compatible, and it is possible to perform an appropriate expansion treatment at the target site such as a stenosed part.

In the present embodiment, the first internal pressure value and the second internal pressure value can be set to desired values, by appropriately setting the amounts of residual strain of the first region 31, the second region 32, and the intermediate section 33. The second internal pressure value may be set on the basis of the pressure intended to act on the target site, and may be, for example, 3 atmospheres (atm). It is preferable to set the first internal pressure value to be sufficiently lower than the second internal pressure value, for example, 0.5 atm, so that the positional deviation prevention effect can be exhibited at an early stage.

Further, in the aforementioned example, the description has been given of a case where the outer diameter in the initial shape is set to be different between the first region 31, the second region 32, and the intermediate section to set amounts of residual strain of both regions different from each other. However, the method for setting different amounts of residual strain for both is not limited thereto. Several methods for generating different amounts of residual strain in the first region 31, the second region 32 and the intermediate section 33 will be described below.

First, by changing an angle formed by the material of the balloon with the folding line 35 between the intermediate section and the other region, the amount of residual strain can be adjusted. That is, in the folding process, as an angle θ1 illustrated in Fig. 11 decreases, the amount of residual strain increases. The angle θ1 can be changed, for example, by changing the shape of the surface in contact with the balloon 3 in the folding member 100 described above.

Further, when the radius of curvature of the top of the wing section increases, the amount of residual strain decreases. Therefore, by forming the folding line 35 at the intermediate section and increasing the radius of curvature of the top of the wing section to such an extent that no ridge line is formed which is clear in the other region, the amount of residual strain at the intermediate section can be relatively increased.

Also, by changing the number of wing sections in the intermediate section and the other region, the amount of residual strain can be adjusted. As the number of wing sections increases, since the number of folding lines 35 and valley sections 37 increases, the amount of residual strain increases.

In addition, when forming the initial shape, if the diameter is reduced without forming the wing sections, a large number of irregular folding lines folded to be weaker than the folding line 35 are formed. Thus, the amount of residual strain decreases.

Therefore, it is possible to relatively increase the amount of residual strain in the intermediate section, also by forming the wing section only in the intermediate section and not forming the wing section in the other regions.

Further, when forming the initial shape, if the wing section is pulled and wound while applying tension, the valley section is strongly bent and the amount of residual strain increases. Therefore, by applying a tension only to the intermediate section or by applying a larger tension to the intermediate section, the amount of residual strain at the intermediate section can be relatively increased.

Furthermore, when incorporating heat treatment at the time of forming the initial shape, the amount of residual strain can be changed, by switching between the presence or absence of heat treatment and the temperature conditions. In general, the amount of residual strain is higher when the heat treatment is performed, and the amount of residual strain is higher when the heat treatment is performed at a higher temperature. Therefore, by applying a heat treatment only to the intermediate section or by setting the temperature of the heat treatment at the intermediate section to be higher than at other regions, the amount of residual strain at the intermediate section can be relatively increased.

Further, when the material of the balloon 3 is partially modified, the amount of residual strain at the intermediate section can be set to be relatively large without changing the process at the time of forming the initial shape for each region. For example, by forming a balloon with a crosslinked polymer and accelerating crosslinking by irradiating only the intermediate section with an electron beam, the rigidity of the intermediate section is relatively enhanced. When a uniform initial shape forming process is performed on this balloon, since the degree of plastic deformation becomes strong at the intermediate section, the amount of residual strain increases.

Each of the above-described methods can be appropriately combined, respectively. Since the amounts of residual strain of each region change complicatedly in combination, for example, it is also possible to set the amount of residual strain of the intermediate section 33 to be relatively large, while setting the outer diameters of the first region 31, the second region 32, and the intermediate section 33 in the initial shape to be the same or substantially the same.

Although the embodiment of the present invention has been described in detail with reference to the drawings, the specific configuration is not limited to this embodiment, and changes in design and the like within the scope not departing from the gist of the present invention are also included.

Further, the constituent elements described in each of the embodiments and each of the modified examples described above can be configured by being appropriately combined.

For example, in the above-described embodiment, the example in which the stylet 6 is inserted into the balloon 3 has been described. However, a configuration in which a sheath having a guide wire lumen and a fluid supply lumen is inserted through the balloon instead of the stylet may be provided. In this case, the guide wire inserted into the guide wire lumen can be made to protrude to the distal end of the balloon, and can be used as a guide for breaking through a site in which a strong constriction occurs, an occlusion part or the like.

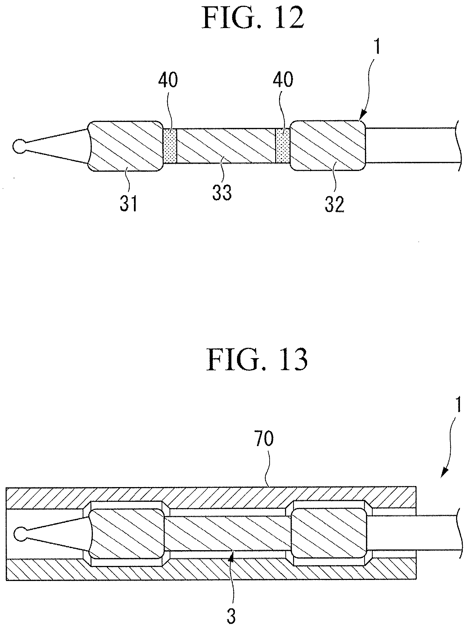

Further, as in the modified example illustrated in Fig. 12, a marker 40 that is visible under endoscope observation or under X-ray fluoroscopy may be provided at a boundary between the first region 31, the second region 32, and the intermediate section 33.

With this configuration, the balloon can be disposed at a more appropriate position with respect to the target site, and the positional deviation prevention effect can be reliably exerted.

As long as the boundary between the intermediate section and another region can be recognized, the marker 40 may be provided in either the intermediate section or the other region.

Further, in the initial shape of the balloon, when the outer diameter difference between the intermediate section and another region is large, a step caused by the outer diameter difference can be used as a marker that can be visually recognized under endoscope observation.

Furthermore, at the time of shipping of the treatment instrument for the endoscope of the present invention, as illustrated in a modified example illustrated in Fig. 13, the balloon 3 may be covered with a cover 70 having an internal space corresponding to the initial shape. In this way, it is possible to suitably maintain the initial shape until usage and to suppress change in amount of residual strain or the like.

Further, in the above embodiment, an example in which the balloon is a semi-compliant type has been described. However, a so-called non-compliant type balloon in which, even if the internal pressure is equal to or higher than the second internal pressure value, the material forming the balloon does not substantially stretch, may be used.

### [Industrial Applicability]

It is possible to stably inflate the balloon at an appropriate position in a luminal organ of a living body and appropriately dilate a stenosed part or an occluded part.

### [Reference Signs List]

1 Treatment instrument for endoscope
2 Sheath
3 Balloon
31 First region
32 Second region
33 Intermediate section
36 Wing section
40 Marker

## Claims

1. A treatment instrument (1) for an endoscope (150), comprising:
a sheath (2); and
a balloon (3) which is provided at a distal end of the sheath (2), the balloon (3) being configured to be expandable to an unfolded inflated shape from a folded initial shape by supplying a fluid, the balloon (3) having a first region (31) and a second region (32) provided at both end portions in a longitudinal direction, and an intermediate section (33) provided between the first region (31) and the second region (32),
**characterized in that**:
in the initial shape of the balloon (3), an amount of residual strain of the intermediate section (33) is larger than the amount of residual strain of the first region (31) and the amount of residual strain of the second region (32),
when an internal pressure of the balloon (3) has a first internal pressure value (P1), the first region (31) and the second region (32) are unfolded to be faster than the intermediate section (33), thereby having a greater diameter than the intermediate section (33),
when the internal pressure of the balloon has a second internal pressure value (P2) greater than the first internal pressure value (P1), the first region (31), the second region (32), and the intermediate section (33) are unfolded to have the inflated shape, and
when the internal pressure of the balloon (3) is greater than a third internal pressure value (P3) greater than the second internal pressure value (P2), a material forming the balloon (3) stretches and expands to be greater than the diameter at the second internal pressure value (P2).

2. The treatment instrument (1) for the endoscope (150) according to claim 1, wherein the balloon (3) is folded to have a plurality of wing sections (36) protruding radially outward in the initial shape, and the wing sections (36) are folded by being wound around an axis of the balloon (3).

3. The treatment instrument (1) for the endoscope (150) according to claim 1, wherein the balloon (3) is configured such that an outer diameter increases up to the second internal pressure value (P2), depending on the inflation due to the progress of the unfolding rather than the expansion due to the stretching of the balloon (3) material, and
at an internal pressure greater than the third internal pressure value (P3), the outer diameter increases, depending on the expansion due to the stretching of the balloon (3) material rather than the inflation due to the progress of the unfolding.

4. The treatment instrument (1) for the endoscope (150) according to claim 1, wherein, in the initial shape, the outer diameter of the intermediate section (33) is smaller than the outer diameters of the first region (31) and the second region (32).

5. The treatment instrument (1) for the endoscope (150) according to claim 1, further comprising:
a marker (40) provided at a boundary between the intermediate section (33), the first region (31), and the second region (32).

6. The treatment instrument (1) for the endoscope (150) according to claim 5, wherein the marker (40) is configured to be visible under an endoscope or X-ray fluoroscopy.

## Patentansprüche

1. Behandlungsinstrument (1) für ein Endoskop (150), das umfasst:
eine Hülse (2); und
einen Ballon (3), der an einem distalen Ende der Hülse (2) vorgesehen ist, wobei der Ballon (3) so konfiguriert ist, dass er durch Zufuhr eines Fluids aus einer gefalteten Ausgangsform in eine entfaltete aufgeblasene Form expandierbar ist, wobei der Ballon (3) einen ersten Bereich (31) und einen zweiten Bereich (32), die an beiden Endabschnitten in einer Längsrichtung vorgesehen sind, und einen Zwischenabschnitt (33) aufweist, der zwischen dem ersten Bereich (31) und dem zweiten Bereich (32) vorgesehen ist,
**dadurch gekennzeichnet, dass**:
in der Ausgangsform des Ballons (3) ein Betrag einer Restdehnung des Zwischenabschnitts (33) größer ist als der Betrag einer Restdehnung des ersten Bereichs (31) und der Betrag einer Restdehnung des zweiten Bereichs (32),
wenn ein Innendruck des Ballons (3) einen ersten Innendruckwert (P1) hat, der erste Bereich (31) und der zweite Bereich (32) so entfaltet werden, dass sie schneller als der Zwischenabschnitt (33) sind, wodurch sie einen größeren Durchmesser als der Zwischenabschnitt (33) haben,
wenn der Innendruck des Ballons einen zweiten Innendruckwert (P2) hat, der größer als der erste Innendruckwert (P1) ist, der erste Bereich (31), der zweite Bereich (32) und der Zwischenabschnitt (33) so entfaltet werden, dass sie die aufgeblasene Form haben, und
wenn der Innendruck des Ballons (3) größer als ein dritter Innendruckwert (P3) ist, der größer als der zweite Innendruckwert (P2) ist, sich ein den Ballon (3) bildendes Material ausdehnt und expandiert, um größer als der Durchmesser bei dem zweiten Innendruckwert (P2) zu sein.

2. Behandlungsinstrument (1) für das Endoskop (150) nach Anspruch 1,
wobei der Ballon (3) so gefaltet ist, dass er eine Mehrzahl von Flügelabschnitten (36) aufweist, die in der Ausgangsform radial nach außen vorstehen, und die Flügelabschnitte (36) gefaltet sind, indem sie um eine Achse des Ballons (3) gewickelt sind.

3. Behandlungsinstrument (1) für das Endoskop (150) nach Anspruch 1,
wobei der Ballon (3) so konfiguriert ist, dass ein Außendurchmesser in Abhängigkeit von der Aufblasung aufgrund des Fortschreitens der Entfaltung und nicht von der Expansion aufgrund der Dehnung des Materials des Ballons (3) bis zum zweiten Innendruckwert (P2) zunimmt, und
sich der Außendurchmesser bei einem Innendruck, der größer als der dritte Innendruckwert (P3) ist, in Abhängigkeit von der Expansion aufgrund der Dehnung des Materials des Ballons (3) und nicht von der Aufblasung aufgrund des Fortschreitens der Entfaltung vergrößert.

4. Behandlungsinstrument (1) für das Endoskop (150) nach Anspruch 1, wobei in der Ausgangsform der Außendurchmesser des Zwischenabschnitts (33) kleiner ist als die Außendurchmesser des ersten Bereichs (31) und des zweiten Bereichs (32).

5. Behandlungsinstrument (1) für das Endoskop (150) nach Anspruch 1, das ferner umfasst:
eine Markierung (40), die an einer Grenze zwischen dem Zwischenabschnitt (33), dem ersten Bereich (31) und dem zweiten Bereich (32) vorgesehen ist.

6. Behandlungsinstrument (1) für das Endoskop (150) nach Anspruch 5, wobei die Markierung (40) so konfiguriert ist, dass sie unter einem Endoskop oder einer Röntgenfluoroskopie sichtbar ist.

## Revendications

1. Instrument de traitement (1) pour un endoscope (150), comprenant :
une gaine (2) ; et
un ballonnet (3) qui est prévu au niveau d'une extrémité distale de la gaine (2), le ballonnet (3) étant configuré pour être extensible jusqu'à une forme gonflée dépliée à partir d'une forme initiale pliée en délivrant un fluide, le ballonnet (3) présentant une première région (31) et une deuxième région (32) prévues au niveau des deux parties d'extrémité dans une direction longitudinale, et une section intermédiaire (33) prévue entre la première région (31) et la deuxième région (32),
**caractérisé en ce que** :
dans la forme initiale du ballonnet (3), une quantité de contrainte résiduelle de la section intermédiaire (33) est supérieure à la quantité de contrainte résiduelle de la première région (31) et à la quantité de contrainte résiduelle de la deuxième région (32),
lorsqu'une pression interne du ballonnet (3) présente une première valeur de pression interne (P1), la première région (31) et la deuxième région (32) sont dépliées pour être plus rapides que la section intermédiaire (33), présentant de ce fait un diamètre supérieur à celui de la section intermédiaire (33),
lorsque la pression interne du ballonnet présente une deuxième valeur de pression interne (P2) supérieure à la première valeur de pression interne (P1), la première région (31), la deuxième région (32) et la section intermédiaire (33) sont dépliées pour avoir la forme gonflée, et
lorsque la pression interne du ballonnet (3) est supérieure à une troisième valeur de pression interne (P3) supérieure à la deuxième valeur de pression interne (P2), un matériau formant le ballonnet (3) s'étire et se dilate pour être plus grand que le diamètre à la deuxième valeur de pression interne (P2).

2. Instrument de traitement (1) pour endoscope (150) selon la revendication 1, dans lequel le ballonnet (3) est plié pour avoir une pluralité de sections d'aile (36) faisant saillie radialement vers l'extérieur dans la forme initiale, et les sections d'aile (36) sont pliées en étant enroulées autour d'un axe du ballonnet (3).

3. Instrument de traitement (1) pour endoscope (150) selon la revendication 1, dans lequel le ballonnet est configuré d'une manière telle qu'un diamètre externe augmente jusqu'à la deuxième valeur de pression interne (P2), en fonction du gonflement dû à la progression du dépliage plutôt que de la dilatation due à l'étirement du matériau de ballonnet (3), et
à une pression interne supérieure à la troisième valeur de pression interne (P3), le diamètre externe augmente, en fonction de la dilatation due à l'étirement du matériau de ballonnet (3) plutôt que du gonflement dû à la progression du dépliage.

4. Instrument de traitement (1) pour endoscope (150) selon la revendication 1, dans lequel, dans la forme initiale, le diamètre externe de la section intermédiaire (33) est inférieur aux diamètres externes de la première région (31) et de la deuxième région (32).

5. Instrument de traitement (1) pour endoscope (150) selon la revendication 1, comprenant en outre :
un marqueur (40) prévue au niveau d'une limite entre la section intermédiaire (33), la première région (31) et la deuxième région (32).

6. Instrument de traitement (1) pour endoscope (150) selon la revendication 5, dans lequel le marqueur (40) est configuré pour être visible sous un endoscope ou une fluoroscopie par rayons X.
